# EUROPEAN PATENT APPLICATION

(11) **EP 2 868 305 A2**
(43) Date of publication of application: **06.05.2015**
(21) Application number: 13750906.3
(22) Date of filing: 25.06.2013
(51) Int. Cl.: A61H 23/02, A61F 7/00

(54) **HEAD FOR A COLD-THERAPY DEVICE**

(30) Priority: 27.06.2012 ES 201230706
(71) Applicant: S.O.R. INTERNACIONAL, S.A., 08192 Sant Quirze Del Vallès (ES)
(72) Inventor: SÁNCHEZ SORIANO, Manuel, E-08192 Sant Quirze del Vallès (Barcelona) (ES)
(74) Representative: Manresa Val, Manuel
(86) International application number: PCT/ES2013/070423
(87) International publication number: WO 2014/001591

(57) **Abstract**

It comprises a Peltier cell with electrical supply means (2) for said Peltier cell, which is located between two conducting plates (3, 4), a first (3) cold conducting plate which will remain facing the patient's skin and a second heat conducting plate (4) in contact with cooling means (5) thereof, mean for measuring the temperature (12) of first conducting plate (3) with said head piece also being connected to an impeller pump and a heat exchanger located outside the head piece and in that first conducting plate (3) is coated with a low thermal conductivity material, and in that it comprises vibration means (7) connected to said first conducting plate (3) which vibrates while cold is transmitted to the patient's skin.

## Description

Head piece for cold therapy apparatus, of the type comprising a Peltier cell with electrical supply means for said Peltier cell, which is located between two conducting plates, a first cold conducting plate that will remain facing the patient's skin and a second heat conducting plate in contact with cooling means therefor, means for measuring the temperature of the first conducting plate, with said head piece being connected also to an impeller pump and a heat exchanger located outside the head piece characterised: in that the first conducting plate is coated with a low thermal conductivity material, and in that it comprises vibration means, connected to said first conducting plate which vibrates as cold is transmitted to the patient's skin.

### BACKGROUND TO THE INVENTION

In the state of the art various machines are known which use cold or cryotherapy, as a method or therapy for reducing obesity and/or cellulite.

So Spanish Patent No. 2300569 (EP1490005) is known, "DEVICES FOR SELECTIVE DISRUPTION OF FATTY TISSUE BY CONTROLLED COOLING", from the year 2003, in the name of THE GENERAL HOSPITAL CORPORATION, which relates to a device for selectively disrupting lipid rich cells in a non-infant human subject by cooling, comprising: cooling means for cooling a local region of the subject's skin to selectively disrupt lipid rich cells of the region, while, concurrently therewith, maintaining the subject's skin at a temperature whereby non-lipid rich cells are not disrupted, wherein the cooling means are adapted to cool the lipid rich cells to a temperature between about - 10°C and about 25 °C, a temperature control unit for controlling the temperature of the cooling means, and temperature measuring means which are adapted to measure the temperature of the subject's skin and/or the temperature in the subject's skin and/or the temperature on the surface of the subject's skin; characterized in that the temperature control unit is further adapted to control the temperature of the cooling means such that the temperature of the subjects skin and/or the temperature in the subject's skin and/or the temperature on the surface of the subject's skin does not fall below a predetermined minimum temperature on the basis of the temperature of the subjects skin and/or the temperature in the subject's skin and/or the temperature on the surface of the subject's skin.

Also from the same company Spanish Patent No. 2359581 (EP1917935) is known, which is a divisional of the above, "METHOD FOR SELECTIVE DISRUPTION OF FATTY TISSUE BY CONTROLLED COOLING", from the year 2003, which relates to a cosmetic treatment method for treating a region selected from one of the abdominal area and the buttock area of a non-infant subject's body to achieve a cosmetically beneficial reduction of subcutaneous adipose tissue, comprising: utilizing at least one feedback device for monitoring temperature and/or crystal formation configured to provide feedback information pertaining to the temperature of the subject's skin or crystal formation; and cooling, based on information received from the at least one feedback device, the subject's skin in a region where subcutaneous adipose tissue reduction is desired by means of cooling said region sufficient to selectively disrupt lipid rich cells therein, and, simultaneously therewith maintaining the subject's skin at a temperature whereby non-lipid rich cells proximate to the cooling means are not disrupted, and whereby non-lipid rich cells surrounding the subcutaneous fatty tissue are not disrupted wherein the lipid rich cells are cooled to a temperature between about -10°C and about 25 °C for a time period from 10 seconds to 30 minutes.

In the two patents above the description refers to the possibility of using a vibration device together with the cooling to increase effectiveness, without explaining how to do this.

The State of the Art includes Spanish Patent No. 2010938 "APARATO DE TERMOTERÁPIA POR FRÍO", from the year 1989, in the name of Mr. Luis CORRAL SANCHEZ, which relates to a cold thermotherapy apparatus provided with a Peltier cell located between two heating plates, one cold and the other receiving heat, with this latter one being cooled by a cooling liquid circuit provided with an impeller pump and a heat exchanger. The unit made up of the Peltier cell and the thermal plates make up a separate cold applicator head piece assembly formed of the apparatus body.

Finally, it is worth mentioning that the same applicant firm sponsored a book entitled "ELECTROESTÉTICA, TEORÍA Y PRÁTICA PARA LA UTILIZACIÓN DE CORRIENTE EN ESTÉTICA" by J.L. VILA BUSQUETS et al., the first edition of which dates from February 1986, where on page 182 thereof is indicates Cryotherapy, Vibrotherapy and Pressotherapy as aesthetic treatments against OBESITY AND/OR CELLULITE.

### BRIEF DESCRIPTION OF THE INVENTION

This application falls within the sector of machines that use cold to perform aesthetic treatments.

The closest document is Spanish Patent No. 2010938. Here it describes a cold generating apparatus for aesthetic treatments, which uses a Peltier cell in its head piece.

The other two patents are based on a similar principle, obtaining registers with a high level of precision for the temperature of the skin epidermis.

The problem with the closest document is that what are known as "thermal shocks" occur, i.e. the apparatus obtains the desired temperature faster than the human body's ability to adapt to said temperature, increasing the feeling of cold and discomfort.

This point is not taken into consideration either in the other two patents mentioned.

So, the inventor has developed a new head piece where the cold conducting plate is coated with a low thermal conductivity cover, in this way, it ensures that the cold be progressive and the human body adapts to it, and it also prevents that the cold conducting plate from touching the patient's skin directly, which prevents burns caused by freezing thermal shocks.

At the same time, to prevent the freezing sensation, the inventor has provided vibration means which cause cold conducting plate vibrate, so that at the same time that the cold is transmitted, a vibration is transmitted which reduces the freezing sensation in the area.

Also, it has been observed that vibrations increase blood flow and toxin elimination.

It has also been demonstrated that this progressive transmission of cold ends up breaking the fatty cells that have just been eliminated by the blood.

The object of this invention is a head piece for cold therapy apparatus, of the type comprising a Peltier cell with electrical supply means for said Peltier cell, which is located between two conducting plates, one first cold conducting plate which will remain facing the patient's skin and a second heat conducting plate in contact with cooling means therefor, means for measuring the temperature of the first conducting plate, with said head piece also being connected to an impeller pump and a heat exchanger located outside the head piece characterised: in that the first conducting plate is coated with a low thermal conductivity material, and in that it comprises vibration means, connected to the said first conducting plate which vibrates as cold is transmitted to the patient's skin.

### BRIEF DESCRIPTION OF THE DRAWINGS

In order to facilitate the explanation this specification includes three sheets of drawings which illustrate a practical embodiment, which is provided as a non-limiting example of the scope of this invention:
Figure 1 is a perspective view of the object of this invention.
Figure 2 is a view of Figure 1 without its casing or chassis thereof, and
Figure 3 is a view of Figure 2 without the cooling means and the second conducting plate, for heat, leaving the Peltier cell on view.

### SPECIFIC EMBODIMENT OF THIS INVENTION

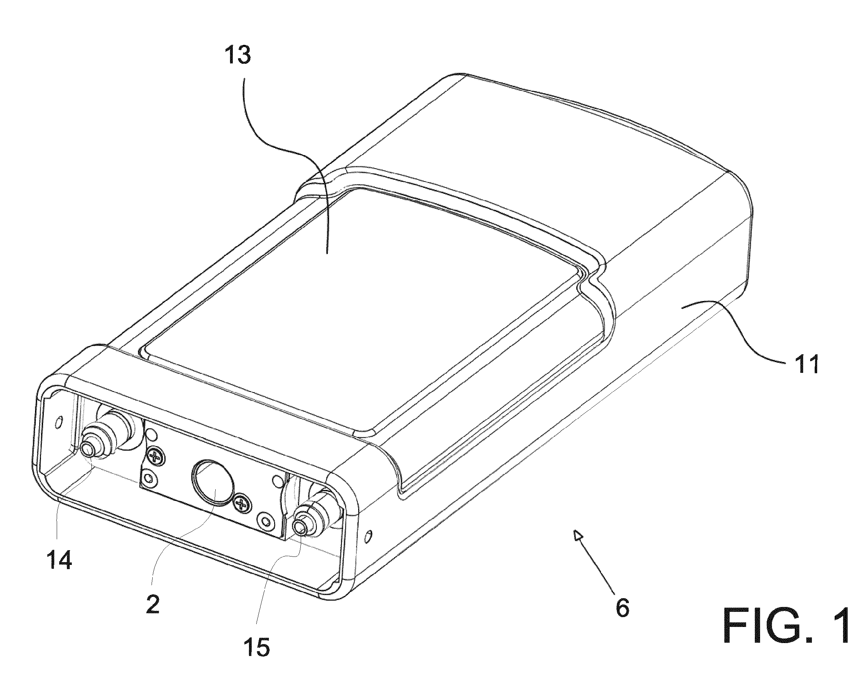

So, Figure 1 illustrates a head piece 6, electrical supply means 2, a chassis or casing 11, a heat dissipating plate 13 and an input duct 14 and another outlet duct 15 for cooling means.

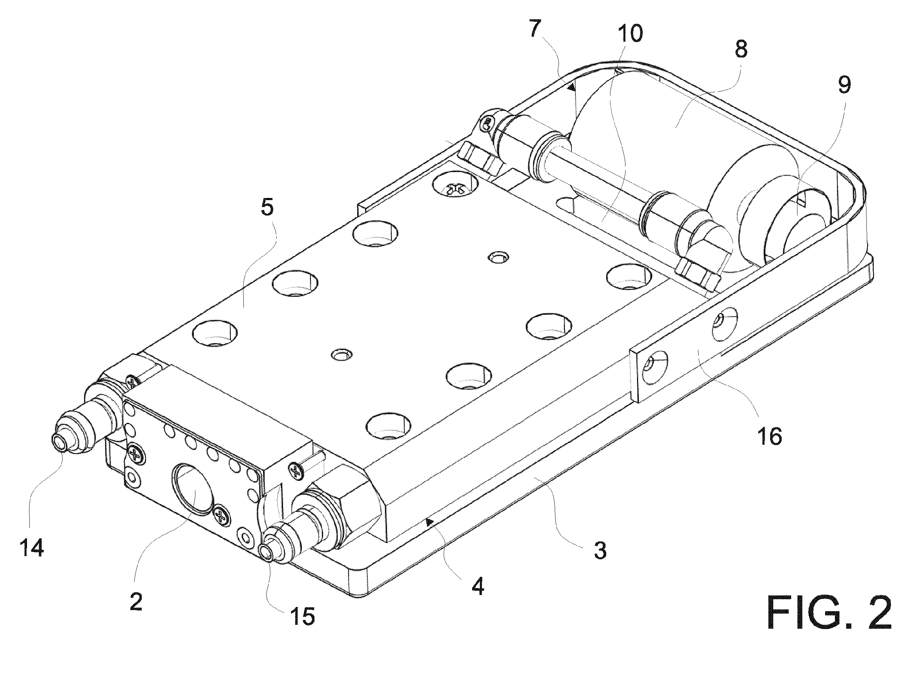

Figure 2 shows the electrical supply means 2, the input duct 14 and the output duct 15 of the cooling means 5, a first conducting plate 3, a second conducting plate 4, vibration means 7 comprising a cylinder 8, an eccentric 9 and a cavity 10 and a reinforcement 16.

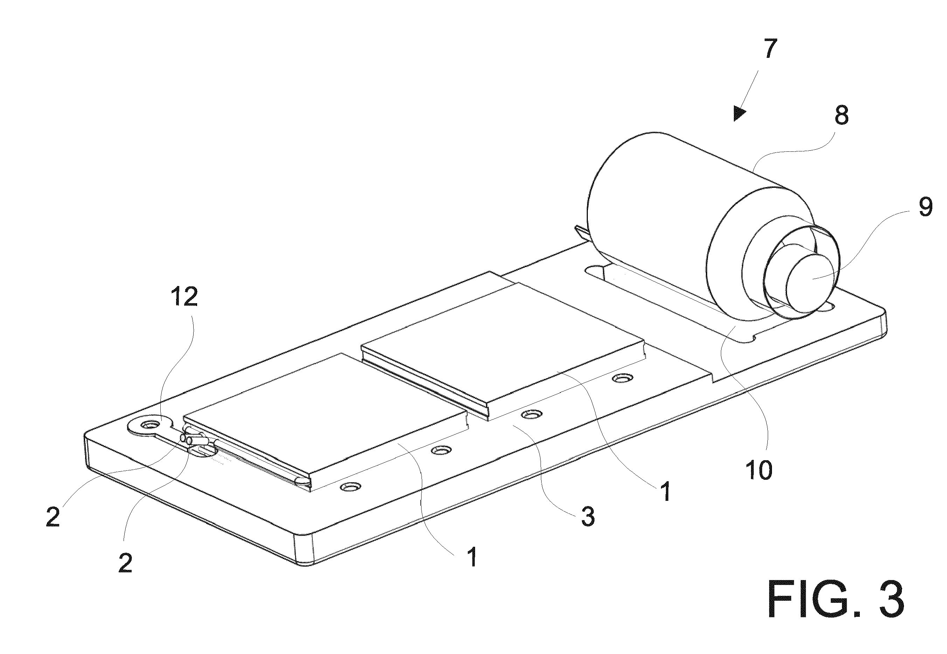

Finally, Figure 3 shows Peltier cells 1, electrical supply means 2, a temperature sensor 12, first conducting plate 3, and vibration means 7 comprising cylinder 8, eccentric 9 and cavity 10.

In this way, in a specific embodiment, before starting, electrical terminals are connected to the connector of the electrical supply means 2 of head piece 6.

The operator then starts the cold therapy apparatus.

When the Peltier cell 1 receives electrical current via connector 2, two types of reactions are produced, one a cold reaction which is directed towards the first conducting plate 3 and another heat reaction which is directed towards second conducting plate 4.

In this embodiment second conducting plate 4 is part of cooling means 5 (Fig. 2). So the heat from second conducting plate 4 is transferred to the cooling liquid that circulates inside cooling means 5 and which exits said means via outlet duct 15. Said cooling liquid is led though an outer tube (not shown) to a heat exchanger (not shown), outside the head piece, where the cooling liquid is cooled, and with said cooling liquid returning to cooling means 5 via input duct 14.

Another configuration option could be such as the one noted in the closest document where second conducting plate 4 and cooling means 5 are two independent bodies, but cooling means 5 are in contact with second conducting plate 4 and this way the transfer of heat from second conducting plate 4 to cooling means 5 is obtained.

The cold that is transmitted to first conducting plate 3, made for example from metal, lowers the temperature of said plate, depending on the treatment, to between -5ºC and 15ºC, with said temperature being controlled by temperature sensor 12.

In particular first conducting plate 3, although it could be the whole head piece 6, is coated with a low conductivity material, such as a plastic material. This is because the inventor wants to avoid thermal shocks, caused by cooling fast of first conducting plate 3 cooling fast, and any contact with the patient's skin producing an uncomfortable sensation for the patient.

Also thanks to said coating the patient does not enter into direct contact with first conducting plate 3, which could cause redness or hyper sensibility.

By ensuring that the user's skin reaches the temperature progressively, said discomfort disappears.

At the same time, vibration means 7 start to operate, so that cylinder 8 rotates on the base of eccentric 9 and knocks cavity 10 in first conducting plate 3, making first conducting plate 3 vibrate by transmitting said vibration to the patient's skin.

Said vibration eliminates the freezing sensation on the patient's skin, and so the patient feels that the quality of the treatment increases.

Other beneficial effects of simultaneously combining cryotherapy and vibrotherapy via first conducting plate 3 is the increased blood flow, thanks both to the vibration and the cold. Also as the blood moves, the toxins are also eliminated from this area.

Finally, the fatty cells that are frozen or partially frozen due to the vibration, separate and are eliminated as a toxin.

Optionally, a heat dissipating plate 13 can be provided on the opposite side to where first conducting plate 3 works, allowing for a heating treatment if necessary.

This utility model describes a new head piece for a cold therapy apparatus. The examples mentioned here are non-limiting with respect to this invention, and therefore said invention can have different applications and/or adaptations, all within the scope of the following claims.

## Claims

1. Head piece for cold therapy apparatus, of the type comprising a Peltier cell (1) with electrical supply means (2) for said Peltier cell, which is located between two conducting plates (3, 4), a first (3) cold conducting plate which will remain facing the patient's skin and a second heat conducting plate (4) in contact with cooling means (5) thereof, means for measuring the temperature (12) of first conducting plate (3), with said head piece (6) also being connected to an impeller pump and a heat exchanger located outside head piece (6) **characterised:**
**in that** first conducting plate (3) is coated with a low thermal conductivity material, and
**in that** it comprises vibration means (7), connected to said first conducting plate (3) which vibrates while cold is transmitted to the patient's skin.

2. Head piece, according to claim 1, **characterised in that** the low thermal conductivity material is a plastic.

3. Head piece, according to claim 1, **characterised in that** vibration means (7) comprise a cylinder (8) moved by an eccentric (9), with said cylinder (8) knocking said first conducting plate (3).

4. Head piece, according to claim 3, **characterised in that** first conducting plate (3) comprises a cavity (10) where cylinder (8) knocks first conducting plate (3).

5. Head piece, according to claim 1, **characterised in that** second conducting plate (4) is part of cooling means (5).

6. Head piece, according to claim 1, **characterised in that** it comprises a heat dissipating plate (13).
